# EUROPÄISCHE PATENTSCHRIFT

(11) **EP 2 478 026 B1**
(45) Veröffentlichungstag und Bekanntmachung des Hinweises auf die Patenterteilung: **18.12.2013**
(21) Anmeldenummer: 10750072.0
(22) Anmeldetag: 04.09.2010
(51) Int. Cl.: A61L 31/10, C08G 18/28, C08G 18/44, C08L 75/04, C09D 175/04, A61L 29/08

(54) **Cyclohexandimethanol-basierte hydrophile polyurethanharnstoffe**
Cyclohexane dimenthanol-based hydrophilic ureae
Urées de polyuréthane hydrophiles à base de cyclo-hexane-di-méthanol

(30) Priorität: 17.09.2009 EP 09011851
(43) Veröffentlichungstag der Anmeldung: 25.07.2012
(73) Patentinhaber: Bayer Materialscience AG, 51368 Leverkusen (DE)
(72) Erfinder: KÖCHER, Jürgen, 40764 Langenfeld (DE); WAMPRECHT, Christian, 41472 Neuss (DE)
(86) Internationale Anmeldenummer: PCT/EP2010/005440
(87) Internationale Veröffentlichungsnummer: WO 2011/032650

(56) Entgegenhaltungen:
- DE-A1- 19 914 885

## Beschreibung

Die vorliegende Erfindung betrifft neuartige Polyurethanharnstoffe, welche zur Herstellung von hydrophilen Beschichtungen auf verschiedensten Substraten verwendet werden können.

Insbesondere im medizinischen Bereich sind hydrophile Beschichtungen von Oberflächen medizintechnischer Geräte von Bedeutung, da ihre Benutzung dadurch stark verbessert werden kann. Das Einsetzen und Verschieben von Urin- oder Blutgefäßkathetern wird dadurch einfacher, dass hydrophile Oberflächen im Kontakt mit Blut oder Urin einen Wasserfilm adsorbieren. Hierdurch wird die Reibung der Katheteroberfläche gegenüber den Gefäßwänden verringert, so dass sich der Katheter leichter einsetzen und bewegen lässt. Auch eine direkte Wässerung der Geräte vor dem Eingriff kann vorgenommen werden, um durch die Bildung eines homogenen Wasserfilms die Reibung zu vermindern. Die betroffenen Patienten haben weniger Schmerzen, und das Risiko von Verletzungen der Gefäßwände wird dadurch reduziert. Darüber hinaus besteht bei der Anwendung von Kathetern immer die Gefahr, dass sich Blutgerinnsel bilden. In diesem Kontext werden im Allgemeinen hydrophile Beschichtungen als hilfreich für antithrombogene Beschichtungen angesehen.

Hydrophile Beschichtungen für medizinische Geräten müssen eine hohe mechanische Belastbarkeit aufweisen. Dies ist insbesondere notwendig, da bei deren Verwendung teils erhebliche mechanische Kräfte einwirken. Dies gilt beispielsweise für Katheter und Stents, wenn sie in Gefäße eingebracht werden. Ein Abplatzen der Beschichtung ist dabei schon unter physiologischen Gesichtspunkten unbedingt zu vermeiden. Zusätzlich muss die Beschichtung aber auch flexibel genug sein, um etwa die Komprimierung eines Stents oder Katheters zu ermöglichen.

Die US 5,589,563 offenbart Beschichtungen mit oberflächenmodifizierten Endgruppen, die auch zur Beschichtung von medizinischen Geräten verwendet werden können. Allerdings weisen diese Beschichtungen noch keine zufriedenstellenden physikalischen Eigenschaften. So ist einerseits deren Hydrophilie nicht ausreichend hoch. Zuletzt sind die Beschichtungen im Allgemeinen nicht ausreichend stabil ausgebildet, d.h. ihre mechanische Belastbarkeit ist zu gering.

Aus der Europäischen Anmeldung Nr. 08153055.2 sind hydrophile Beschichtungen aus Polyurethanharnstoffen bekannt, die auf eine speziellen Kombination von Polycarbonatpolyolen als Aufbaukomponenten und Copolymeren aus Ethylen- und Propylenoxid als Endgruppen basieren. Auch diese Beschichtungen genügen in ihren mechanischen Eigenschaften noch nicht allen Anforderungen.

Es war daher Aufgabe der Erfindung, einen Beschichtungsgrundstoff bereit zu stellen, der zur Herstellung von hydrophilen, mechanisch hoch belastbaren Beschichtungen insbesondere auf medizinischen Geräten wie Stents oder Kathetern verwendet werden kann.

Diese Aufgabe ist durch einen Polyurethanharnstoff gelöst, der mit wenigstens einer Copolymereinheit aus Polyethylenoxid und Polypropylenoxid terminiert ist und der wenigstens eine Struktureinheit der Formel (I) 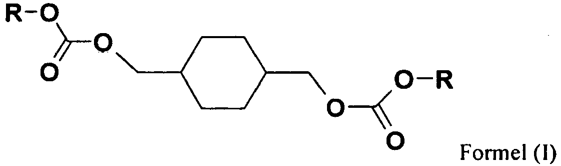 aufweist, die über wenigstens eine Bindung R mit der Polymerkette verknüpft ist.

Die mit Hilfe des erfindungsgemäßen Polyurethanharnstoffs erhältlichen Beschichtungen zeichnen sich durch eine hohe Hydrophilie und eine hohe mechanische Belastbarkeit aus.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 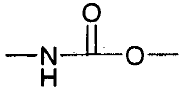 und
(b) mindestens eine Harnstoffgruppen enthaltende Wiederholungseinheit 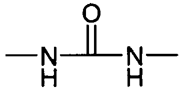 aufweisen.

Die erfindungsgemäßen Polyurethanharnstoffe weisen bevorzugt keine ionische oder ionogene Modifizierung auf. Hierunter wird im Rahmen der vorliegenden Erfindung verstanden, dass sie im Wesentlichen keine ionischen Gruppen, wie insbesondere Sulfonat-, Carboxylat-, Phosphat- und Phosphonatgruppen aufweisen.

Unter dem Begriff "im Wesentlichen keine ionische Gruppen" wird im Rahmen der vorliegenden Erfindung verstanden, dass die resultierenden Beschichtung des Polyurethanharnstoffs ionische Gruppen mit einem Anteil von im Allgemeinen höchstens 2,50 Gew.-%, insbesondere höchstens 2,00 Gew.-%, vorzugsweise höchstens 1,50 Gew.-%, besonders bevorzugt höchstens 1,00 Gew.-%, speziell höchstens 0,50 Gew.-%, noch spezieller keine ionische Gruppen aufweist. Damit ist insbesondere bevorzugt, dass der Polyurethanharnstoff keine ionischen Gruppen aufweist, da hohe Konzentration an Ionen in organischer Lösung dazu führen, dass das Polymer nicht mehr ausreichend löslich ist und somit keine stabilen Lösungen erhalten werden können. Falls der Polyurethanharnstoff ionische Gruppen aufweist, handelt es sich bevorzugt um Carboxylate und Sulfonate.

Die Polyurethanharnstoffe sind bevorzugt im Wesentlichen lineare Moleküle, können jedoch auch verzweigt sein, was jedoch weniger bevorzugt ist. Unter im Wesentlichen linearen Molekülen versteht man im Rahmen der vorliegenden Erfindung leicht anvernetzte Systeme, wobei die zugrundeliegende Polycarbonatpolyolkomponente bevorzugt eine mittlere Hydroxylfunktionalität von bevorzugt 1,7 bis 2,3, besonders bevorzugt 1,8 bis 2,2, ganz besonders bevorzugt 1,9 bis 2,1, aufweist.

Das zahlenmittlere Molekulargewicht der Polyurethanharnstoffe beträgt vorzugsweise 1000 bis 200000 g/mol, besonders bevorzugt von 3000 bis 100000 g/mol. Dabei wird das zahlenmittlere Molekulargewicht gegen Polystyrol als Standard in Dimethylacetamid bei 30 °C gemessen.

Die Polyurethanharnstoffe können durch Umsetzung von Aufbaukomponenten hergestellt werden, die mindestens eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente e) umfassen.

Daher ist ein Verfahren zur Herstellung der erfindungsgemäßen Polyurethanharnstoffe ebenfalls ein Gegenstand der Erfindung, bei dem eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente miteinander umgesetzt werden.

Komponente a) umfasst wenigstens ein Polycarbonatpolyol a1), welches durch Umsetzung von Kohlensäurederivaten, wie Diphenylcarbonat, Dimethylcarbonat oder Phosgen mit difunktionellen Alkoholen der Formel (II) 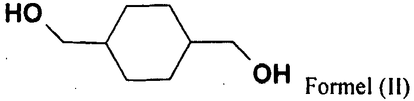 erhalten wird.

Zur Herstellung werden in einem Druckreaktor bei erhöhter Temperatur 1,4-Cyclohexandimethanol mit Diphenylcarbonat, Dimethylcarbonat oder Phosgen umgesetzt. Bevorzugt ist die Umsetzung mit Dimethylcarbonat. Im Falle der Verwendung von Dimethylcarbonat wird das Spaltprodukt Methanol im Gemisch mit überzähligem Dimethylcarbonat per Destillation entfernt.

Die Herstellung der Polycarbonatpolyole mittels Dimethylcarbonat erfolgt bei Temperaturen von bis zu 240 °C, vorzugsweise bis zu 220 °C und besonders bevorzugt bis zu 200 °C. Es kann sowohl drucklos, als auch unter einem Druck von bis zu 15 bar, vorzugsweise bis zu 10 bar und besonders bevorzugt bis zu 5 bar gearbeitet werden. Weiterhin findet die Herstellung der Polycarbonate in manchen Teilschritten (Entwässerung, Destillation) auch bei einem Vakuum von < 500 mbar, vorzugsweise < 100 mbar und besonders bevorzugt < 20 mbar statt.

Die Polycarbonatpolyole a1) auf Basis von Diolen der Formel (II) haben durch die OH-Zahl bestimmte Molekulargewicht von vorzugsweise 200 bis 10000 g/mol, besonders bevorzugt 300 und 8000 g/mol und ganz besonders bevorzugt 400 bis 6000 g/mol.

Bevorzugt ist Komponente a) eine Mischung aus vorgenannten Polycarbonatpolyolen a1) auf Basis von Diolen der Formel (II) und weiteren Polycarbonatpolyolen a2).

Solche weiteren Polycarbonatpolyole a2) haben bevorzugt mittlere Hydroxylfunktionalitäten von 1,7 bis 2,3, besonders bevorzugt von 1,8 bis 2,2, ganz besonders bevorzugt von 1,9 bis 2,1.

Ferner weisen die Polycarbonatpolyole a2) durch die OH-Zahl bestimmte Molekulargewichte von vorzugsweise 400 bis 6000 g/mol, besonders bevorzugt von 500 bis 5000 g/mol, insbesondere von 600 bis 3000 g/mol auf, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Die Polycarbonatpolyole a2) enthalten bevorzugt 40 bis 100 Gew.-% Hexandiol, bevorzugt 1,6-Hexandiol und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden, als Aufbaukomponenten. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate a2) auf Basis von Hexandiol-1,6, sowie modifizierend wirkenden Co-Diolen wie z. B. Butandiol-1,4 oder auch von ε-Caprolacton. Weitere bevorzugte Polycarbonatdiole a2) sind solche auf Basis von Mischungen aus Hexandiol-1,6 und Butandiol-1,4.

In einer bevorzugten Ausführungsform wird in a) eine Mischung aus den Polycarbonatpolyolen a1) und solchen Polycarbonatpolyolen a2) auf Basis von Hexandiol-1,6, Butandiol-1,4 oder deren Mischungen eingesetzt.

Im Fall von Mischungen der Bestandteile a1) und a2) beträgt der Anteil von a1) an der Mischung bevorzugt mindestens 5 mol%, besonders bevorzugt mindestens 10 mol%, bezogen auf die gesamte Molmenge an Polycarbonat.

Die Polyurethanharnstoffe weisen ferner Einheiten auf, welche auf mindestens ein Polyisocyanat als Aufbaukomponente b) zurückgehen.

Als Polyisocyanate b) können alle dem Fachmann bekannten aromatischen, araliphatischen; aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden. Sie können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiiosocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methyl-cyclohexylisocyanat (IMCI) und/oder 4,4'-Bis-(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion- und/oder Oxadiazintrionstruktur mit mehr als zwei NCO-Gruppen.

Die Menge an Bestandteil b) bei der Herstellung der Polyurethanharnstoffe beträgt vorzugsweise 1,0 bis 3,5 mol, besonders bevorzugt 1,0 bis 3,3 mol, insbesondere 1,0 bis 3,0 mol, jeweils bezogen auf die Menge der Verbindungen der Komponente a).

Die Polyurethanharnstoffe weisen Einheiten auf, welche auf ein Copolymer aus Polyethylenoxid und Polypropylenoxid als Aufbaukomponente c) zurückgehen. Diese Copolymereinheiten liegen als Endgruppen in dem Polyurethanharnstoff vor und bewirken eine besonders vorteilhafte Hydrophilierung.

Solche nichtionisch hydrophilierenden Verbindungen c) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Die Alkylenoxide Ethylenoxid und Propylenoxid können in beliebiger Reihenfolge oder auch im Gemisch bei der Alkoxylierungsreaktion eingesetzt werden können.

Bei den Polyalkylenoxidpolyetheralkoholen handelt es sich um gemischte Polyalkylenoxidpolyether aus Ethylenoxid und Propylenoxid, deren Alkylenoxideinheiten vorzugsweise zu mindestens 30 mol-%, besonders bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten bezogen auf den Gesamtanteil an Alkylenoxideinheiten aufweisen.

Das zahlenmittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil c) bei der Herstellung der Polyurethanharnstoffe beträgt vorzugsweise 0,01 bis 0,5 mol, besonders bevorzugt 0,02 bis 0,4 mol, insbesondere 0,04 bis 0,3 mol, jeweils bezogen auf die Menge der Verbindungen der Komponente a).

Es konnte gezeigt werden, dass sich die Polyurethanharnstoffe mit Endgruppen, die auf gemischten Polyoxyalkylenether aus Polyethylenoxid und Polypropylenoxid basieren, insbesondere dazu eignen, Beschichtungen mit einer hohen Hydrophilie zu erzeugen.

Die Polyurethanharnstoffe können Einheiten aufweisen, welche auf mindestens ein Diamin oder ein Aminoalkohol als Aufbaukomponente zurückgehen und als sogenannte Kettenverlängerer d) dienen.

Solche Kettenverlängerer sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4'-Diaminodicyclohexylmethan, Dimethylethylendiamin, Hydrazin, A-dipinsäuredihydrazid, 1,4-Bis(aminomethyl)cyclohexan, 4,4'-Diamino-3,3'-dimethyldicyclohexyl-methan und andere (C₁ - C₄)-Di- und Tetraalkyldicyclo-hexylmethane, z. B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-Ethylaminopropan, 3-Amino-1-cyclohexylaminopropan, 3-Amino-1- Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil d) der Polyurethanharnstoffe kann bei deren Herstellung als Kettenverlängerer eingesetzt werden.

Die Menge an Bestandteil d) bei der Herstellung der Polyurethanharnstoffe beträgt vorzugsweise 0,1 bis 1,5 mol, besonders bevorzugt 0,2 bis 1,3 mol, insbesondere 0,3 bis 1,2 mol, jeweils bezogen auf die Menge der Verbindungen der Komponente a).

In einer weiteren Ausführungsform umfassen die Polyurethanharnstoffe zusätzliche Einheiten, welche auf mindestens ein weiteres Polyol als Aufbaukomponente zurückgehen.

Die zum Aufbau der Polyurethanharnstoffe eingesetzten weiteren niedermolekularen Polyole e) bewirken in der Regel eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z.B. α-Hydroxybutyl-ε-hydroxycapronsäureester, ω-Hydroxyhexyl-γ-hydroxybuttersäure-ester, Adipinsäure-(ß-hydroxyethyl)ester oder Terephthalsäure-bis(ß-hydroxyethyl)-ester können verwendet werden.

Die Menge an Bestandteil e) bei der Herstellung der Polyurethanharnstoffe beträgt vorzugsweise 0,05 bis 1,0 mol, besonders bevorzugt 0,05 bis 0,5 mol, insbesondere 0,1 bis 0,5 mol, jeweils bezogen auf die Menge der Verbindungen der Komponente a).

Die Umsetzung der isocyanathaltigen Komponente b) mit den hydroxy- oder aminfunktionellen Verbindungen a), c), d) und gegebenenfalls e) erfolgt üblicherweise unter Einhaltung eines leichten NCO-Überschusses gegenüber den reaktiven Hydroxy- oder Aminverbindungen. Am Endpunkt der Reaktion durch Erreichen einer Zielviskosität verbleiben immer noch Reste an aktivem Isocyanat. Diese Reste müssen im Fall einer Lösungsmittelpolymerisation blockiert werden, damit nicht eine Reaktion mit großen Polymerketten stattfindet. Eine solche Reaktion führt zur dreidimensionalen Vernetzung und Vergelung des Ansatzes. Die Verarbeitung einer solchen Polyurethan-harnstoff-Lösung ist nicht mehr möglich. Üblicherweise enthalten die Ansätze hohe Mengen an Alkoholen. Diese Alkohole blockieren innerhalb von mehreren Stunden Stehen oder Rühren des Ansatzes bei Raumtemperatur die noch verbliebenen Isocyanatgruppen. Im Fall der Herstellung einer wässrigen Polyurethandispersion werden die überschüssigen Isocananatgruppen üblicherweise durch das zugegebene Wasser hydrolysiert. Man kann aber die überschüssigen Isocanatgruppen auch vor der Zugabe des Wassers oder vor der Alkoholyse durch die Lösungsmittelalkohole auch durch gezielte Zugabe einer Komponente f) blockieren.

Wurde bei der Herstellung der Polyurethanharnstoffe in organischer Lösung oder in wässriger Dispersion der Restisocyanatgehalt blockiert, weisen diese auch Monomere f) als Aufbaukomponenten auf, die sich jeweils an den Kettenenden befinden und diese abschließen.

Diese Aufbaukomponenten leiten sich zum einen von monofunktionellen, mit NCO-Gruppen reaktiven Verbindungen ab, wie Monoaminen, insbesondere mono-sekundären Aminen oder Monoalkoholen. Genannt seien hier beispielsweise Ethanol, n-Butanol, Ethylenglykol-monobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine f) im Wesentlichen dazu verwendet werden, den NCO-Überschuss zu vernichten, hängt die erforderliche Menge im Wesentlichen von der Menge des NCO-Überschusses ab und kann nicht allgemein spezifiziert werden.

Bevorzugt wird während der Synthese auf diese Bausteine verzichtet. Noch nicht umgesetztes Isocyanat wird im Fall der organischen Polyurethanharnstofflösungen dabei vorzugsweise durch die in sehr großen Konzentrationen enthaltenen Lösungsmittelalkohole zu terminalen Urethanen umgesetzt. Im Fall der Herstellung einer wässrigen Dispersion werden die überschüssigen Isocyanatgruppen üblicherweise durch das zugegebene Dispergierwasser hydrolysiert.

Zur Herstellung der Polyurethanharnstoffe in organischer Lösung werden die Polycarbonatpolyolkomponente a), das Polyisocyanat, der monofunktionelle Polyetheralkohol und gegebenenfalls das Polyol in der Schmelze oder in Lösung miteinander umgesetzt, bis alle Hydroxylgruppen verbraucht sind.

Die dabei verwendete Stöchiometrie zwischen den einzelnen an der Umsetzung beteiligten Aufbaukomponenten ergibt sich aus den zuvor erwähnten Mengenverhältnissen.

Die Umsetzung erfolgt bei einer Temperatur von vorzugsweise 60 bis 110 °C, besonders bevorzugt von 75 bis 110 °C, insbesondere von 90 bis 110 °C, wobei Temperaturen um 110 °C aufgrund der Geschwindigkeit der Umsetzung bevorzugt sind. Höhere Temperaturen können ebenfalls angewendet werden, allerdings besteht dann im Einzelfall und in Abhängigkeit der einzelnen verwendeten Bestandteile das Risiko, dass Zersetzungsprozesse und Verfärbungen in dem entstehenden Polymer auftreten.

Bei dem Prepolymer aus Isocyanat und allen Hydroxylgruppen aufweisenden Komponenten ist die Umsetzung in Schmelze bevorzugt, allerdings besteht die Gefahr, dass es zu hohen Viskositäten der ausreagierten Gemische kommt. In diesen Fällen empfiehlt es sich auch, Lösungsmittel hinzuzugeben. Es sollte aber möglichst nicht mehr als ungefähr 50 Gew.-% Lösungsmittel enthalten sein, da andernfalls die Verdünnung die Reaktionsgeschwindigkeit deutlich verlangsamt.

Bei der Umsetzung von Isocyanat und den Hydroxylgruppen aufweisenden Komponenten kann die Reaktion in der Schmelze in einem Zeitraum von 1 Stunde bis 24 Stunden erfolgen. Geringe Zugabe von Lösungsmittelmengen führen zu einer Verlangsamung, wobei die Umsetzungszeiträume jedoch in den gleichen Zeiträumen liegen.

Die Reihenfolge der Zugabe bzw. Umsetzung der einzelnen Bestandteile kann von der zuvor angegebenen Reihenfolge abweichen. Dieses kann insbesondere dann von Vorteil sein, wenn die mechanischen Eigenschaften der resultierenden Beschichtungen verändert werden sollen. Wenn man beispielsweise alle Hydroxylgruppen aufweisenden Komponenten gleichzeitig umsetzt, entsteht ein Gemisch aus Hart- und Weichsegmenten. Wenn man beispielsweise das niedermolekulare Polyol nach der Polycarbonatpolyolkomponente zugibt, erhält man definierte Blöcke, was andere Eigenschaften der resultierenden Beschichtungen mit sich bringen kann. Die vorliegende Erfindung ist somit nicht auf eine bestimmte Reihenfolge der Zugabe bzw. Umsetzung der einzelnen Bestandteile der Polyurethanbeschichtung beschränkt.

Im Fall der Herstellung eines Polyurethanharnstoffes in organischer Lösung wird dann weiteres Lösungsmittel zugesetzt und das gegebenenfalls gelöste Kettenverlängerungsdiamin bzw. der gelöste Kettenverlängerungsaminoalkohol (Aufbaukomponente (d)) zugegeben.

Die weitere Zugabe des Lösungsmittels erfolgt vorzugsweise schrittweise, um die Reaktion nicht unnötig zu verlangsamen, was bei einer vollständigen Zugabe der Lösungsmittelmenge beispielsweise am Anfang der Umsetzung passieren würde. Ferner ist man bei einem hohen Gehalt an Lösungsmittel zum Beginn der Reaktion an eine im Verhältnis niedrige Temperatur gebunden, welche von der Art des Lösungsmittels zumindest mitbestimmt wird. Auch dieses führt zu einer Verlangsamung der Reaktion.

Nach Erreichen der Zielviskosität können die noch verbleibenden Reste an NCO durch ein monofunktionelles aliphatisches Amin blockiert werden. Bevorzugt blockiert man die noch verbliebenen Isocanatgruppen durch Umsetzung mit den im Lösungsmittelgemisch enthaltenen Alkoholen.

Als Lösungsmittel für die Herstellung und die Anwendung der erfindungsgemäßen Polyurethanharnstoff-Lösungen kommen alle denkbaren Lösungsmittel und Lösungsmittelgemische wie Dimethylformamid, N-Methylacetamid, Tetramethylharnstoff, N-Methylpyrrolidon, aromatische Lösungsmittel wie Toluol, lineare und cyclische Ester, Ether, Ketone und Alkohole in Frage. Beispiele für Ester und Ketone sind beispielsweise Ethylacetat, Butylacetat, Aceton, γ-Butyrolacton, Methylethylketon und Methylisobutylketon.

Bevorzugt sind Mischungen aus Alkoholen mit Toluol. Beispiele für die Alkohole, die gemeinsam mit dem Toluol verwendet werden, sind Ethanol, n-Propanol, iso-Propanol und 1-Methoxy-2-propanol.

Im Allgemeinen wird in der Umsetzung so viel Lösungsmittel eingesetzt, dass man ungefähr 10 bis 50 gew.-%ige Lösungen, bevorzugt ungefähr 15 bis 45 gew.-%ige Lösungen und besonders bevorzugt ungefähr 20 bis 40 gew.-%ige Lösungen erhält.

Der Feststoffgehalt der Polyurethanharnstofflösungen liegt im Allgemeinen im Bereich von 5 bis 60 Gew.-%, vorzugsweise von 10 bis 40 Gew.-%. Für Beschichtungsversuche können die Polyurethanlösungen beliebig mit Toluol/Alkohol-Gemischen verdünnt werden, um die Dicke der Beschichtung variabel einstellen zu können. Alle Konzentrationen von 1 bis 60 Gew.-% sind möglich, bevorzugt sind Konzentrationen im Bereich von 1 bis 40 Gew.-%. Dabei können beliebige Schichtdicken wie beispielsweise einige 100 nm bis hinauf zu einigen 100 µm erreicht werden, wobei im Rahmen der vorliegenden Erfindung auch höhere und geringere Dicken möglich sind. Die erfindungsgemäßen Polyurethanharnstoffdispersionen werden dabei vorzugsweise nach dem sogenannten Aceton-Verfahren hergestellt. Für die Herstellung der Polyurethanharnstoffdispersion nach diesem Aceton-Verfahren werden üblicherweise die Bestandteile a), c) und e), die keine primären oder sekundären Aminogruppen aufweisen dürfen und die Polyisocyanatkomponente b) zur Herstellung eines isocyanatfunktionellen Polyurethan-Prepolymers ganz oder teilweise vorgelegt und gegebenenfalls mit einem mit Wasser mischbaren, aber gegenüber Isocyanatgruppen inerten Lösungsmittel verdünnt und auf Temperaturen im Bereich von 50 bis 120 °C aufgeheizt. Zur Beschleunigung der Isocyanatadditionsreaktion können die in der Polyurethan-Chemie bekannten Katalysatoren eingesetzt werden, beispielsweise Dibutylzinndilaurat. Bevorzugt ist die Synthese ohne Katalysator.

Geeignete Lösungsmittel sind die üblichen aliphatischen, ketofunktionellen Lösungsmittel wie z.B. Aceton, Butanon, die nicht nur zu Beginn der Herstellung, sondern gegebenenfalls in Teilen auch später zugegeben werden können. Bevorzugt sind Aceton und Butanon. Andere Lösungsmittel wie z.B. Xylol, Toluol, Cyclohexan, Butylacetat, Methoxypropylacetat, Lösungsmittel mit Ether- oder Estereinheiten können ebenfalls eingesetzt und ganz oder teilweise abdestilliert werden oder vollständig in der Dispersion verbleiben.

Anschließend werden die gegebenenfalls zu Beginn der Reaktion noch nicht zugegebenen Bestandteile von c) und e) zudosiert.

In einer bevorzugten Weise wird das Prepolymer ohne Lösungsmittelzusatz hergestellt und erst für die Kettenverlängerung mit einem geeigneten Lösungsmittel, vorzugsweise Aceton, verdünnt.

Die Umsetzung zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder Butanon gelöst.

Anschließend werden mögliche NH₂-, NH-funktionelle und/oder OH-funktionelle Komponenten mit den noch verbliebenen Isocyanatgruppen umgesetzt. Diese Kettenverlängerung/-terminierung kann dabei entweder in Lösungsmittel vor dem Dispergieren, während des Dispergierens oder in Wasser nach dem Dispergieren durchgeführt werden. Bevorzugt wird die Kettenverlängerung vor der Dispergierung in Wasser durchgeführt.

Werden zur Kettenverlängerung Verbindungen entsprechend der Definition von d) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung eingesetzten Verbindungen zu freien NCO-Gruppen des Prepolymers liegt vorzugsweise zwischen 40 bis 150 %, besonders bevorzugt zwischen 50 bis 120 %, insbesondere zwischen 60 bis 120 %.

Die aminischen Komponenten d) können gegebenenfalls in wasser- oder lösungsmittelverdünnter Form im erfindungsgemäßen Verfahren einzeln oder in Mischungen eingesetzt werden, wobei grundsätzlich jede Reihenfolge der Zugabe möglich ist.

Wenn die restlichen verbliebenen Isocyanatgruppen nicht durch das Dispergierwasser hydrolysiert werden sollen, kann man vor Zugabe des Dispergierwassers zur Blockierung der Isocyanatgruppen hier eine ausreichende Menge der Komponente f) zugeben.

Wenn Wasser oder organische Lösungsmittel als Verdünnungsmittel mitverwendet werden, so beträgt der Verdünnungsmittelgehalt bevorzugt 70 bis 95 Gew.-%.

Die Herstellung der Polyurethandispersion aus den Prepolymeren erfolgt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den Prepolymerlösungen gerührt. Bevorzugt wird das Wasser in das gelöste Prepolymer gegeben.

Das in den Dispersionen nach dem Dispergierschritt noch enthaltene Lösungsmittel wird üblicherweise anschließend destillativ entfernt. Eine Entfernung bereits während der Dispergierung ist ebenfalls möglich.

Der Feststoffgehalt der Polyurethandispersion nach der Synthese liegt im Bereich von 20 bis 70 Gew.-%, bevorzugt 20 bis 65 Gew.-%. Für Beschichtungsversuche können diese Dispersionen beliebig mit Wasser verdünnt werden, um die Dicke der Beschichtung variabel einstellen zu können. Alle Konzentrationen von 1 bis 60 Gew.-% sind möglich, bevorzugt sind Konzentrationen im Bereich 1 bis 40 Gew.-%.

Die Polyurethanharnstoffe können ferner für den jeweils angestrebten Einsatzzweck übliche Bestandteile und Additive enthalten.

Ein Beispiel hierfür sind pharmakologische Wirkstoffe, Arzneimittel und Additive, die die Freisetzung von pharmakologischen Wirkstoffen fördern ("drug-eluting-Additive").

Pharmakologische Wirkstoffe bzw. Arzneimittel, die in den erfindungsgemäßen Beschichtungen auf den medizinischen Geräten verwendet werden können, sind beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklusregulierende Mittel, genetische Mittel, Hormone, sowie deren Homologe, Derivate, Fragmente, pharmazeutische Salze und Kombinationen davon.

Spezifische Beispiele solcher pharmakologischen Wirkstoffe bzw. Arzneimittel schließen somit thromboresistente (nichtthrombogene) Mittel bzw. andere Mittel zur Unterdrückung einer akuten Thrombose, Stenose oder späten Re-Stenose der Arterien wie beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw ein.

Ein Wachstumsfaktor kann ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Der pharmakologische Wirkstoff bzw. das Arzneimittel kann auch aus einem Vasodilatator bestehen, um Vasospasmus entgegen zu wirken. Ein Beispiel ist hier ein Antispasmusmittel wie Papaverin. Das Arzneimittel kann ein vasoaktives Mittel wie Calciumantagonisten an sich, oder α- und β-adrenergische Agonisten oder Antagonisten sein. Zusätzlich kann das therapeutische Mittel ein biologisches Haftmittel wie Cyanoacrylat in medizinischer Qualität oder Fibrin, das beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumor-stelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotische Mittel wie Penicilline, Cephalosporine, Vacomycine, Aminoglycoside, Quinolone, Polymyxine, Erythromycine; Tetracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon;
(d) antivrale Mittel wie Amantadin, Rimantadin, Rabavirin, Idoxuridin, Vidarabin, Trifluridin, Acyclovir, Ganciclocir, Zidovudin, Phosphonoformate, Interferone, deren Homologe, Analoge, Fragmente, Derivate, pharmazeutische Salze und Mischungen davon; und
(e) entzündungshemmende Mittel wie beispielsweise Ibuprofen, Dexamethason oder Methylprednisolon.

Zur Erzeugung von Oberflächen mit bewuchshemmenden Eigenschaften können die erfindungsgemäßen Beschichtungszusammensetzungen die aus dem Stand der Technik bekannten bewuchshemmenden Wirkstoffe enthalten. Deren Anwesenheit verstärkt in der Regel die bereits hervorragenden bewuchshemmenden Eigenschaften der mit den Beschichtungszusammensetzungen selbst erzeugten Oberflächen.

Weitere Zusätze wie beispielsweise Antioxidantien oder Pigmente können ebenfalls verwendet werden. Darüber hinaus können gegebenenfalls noch weitere Zusätze wie Griffhilfsmittel, Farbstoffe, Mattierungsmittel, UV-Stabilisatoren, Lichtstabilisatoren, Hydrophobierungsmittel und/oder Verlaufshilfsmittel verwendet werden.

Die erfindungsgemäßen Polyurethanharnstoffe können dazu verwendet werden, eine Beschichtung zum Beispiel auf einem medizinischen Gerät zu bilden.

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Harnleiterkathetern; zentralvenöse Katheter; venöse Kathetern oder Einlass- bzw. Auslass-Kathetern; Dilationsballons; Kathetern für die Angioplastie und die Biopsie; Kathetern, die für das Einbringen eines Stents, eines Propf- oder eines Kavafilters verwendet werden; Ballonkathetern oder andere dehnbare medizinische Geräte; Endoskope; Larnygoskope; Trachealgeräte wie Endotrachealschläuche, Atemgeräte und andere Trachealabsauggeräte; bronchoalveolare Spülungskathetern; Kathetern, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Polyurethanharnstoffe zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mittel für Stents oder für Ballonoberflächen oder für Kontrazeptiva.

Üblicherweise sind die medizinischen Geräte aus Kathetern, Endoskopen, Laryngoskopen, Endotrachealschläuchen, Ernährungsschläuchen, Führungsstäben, Stents, und andere Implantate ausgewählt.

Zusätzlich zu den hydrophilen Eigenschaften der Verbesserung der Gleitfähigkeit zeichnen sich die erfindungsgemäß vorgesehenen Beschichtungszusammensetzungen auch durch eine hohe Blutverträglichkeit aus. Hierdurch ist auch ein Arbeiten mit diesen Beschichtungen besonders im Blutkontakt vorteilhaft. Die Materialien zeigen im Vergleich zu Polymeren des Standes der Technik eine reduzierte Gerinnungsneigung im Blutkontakt.

Wirkstofffreisetzende Systeme basierend auf den erfindungsgemäßen hydrophilen Beschichtungsmaterialien sind auch außerhalb der Medizintechnik denkbar, beispielsweise für Anwendungen im Pflanzenschutz als Trägermaterial für Wirkstoffe. Die gesamte Beschichtung kann dann als wirkstofffreisetzendes System betrachtet werden und beispielsweise zur Beschichtung von Saatgut (Samenkörner) eingesetzt werden. Durch die hydrophilen Eigenschaften der Beschichtung kann der enthaltene Wirkstoff im feuchten Erdreich austreten und seine bestimmungsgemäße Wirkung entfalten ohne dass die Keimfähigkeit des Saatgutes beeinträchtigt wird. Im trockenen Zustand bindet das Beschichtungsmittel den Wirkstoff jedoch sicher an das Saatgut, so dass beispielsweise beim Einschießen des Samenkorns mit der Ausbringmaschine in den Boden der Wirkstoff nicht abgelöst wird, wodurch er unerwünschte Wirkungen z.B. auf die vorhandene Fauna entfalten könnte (Bienengefährdung durch Insektizide, die an sich den Insektenbefall des Samenkorns im Boden verhindern sollen).

Über die Anwendung als Beschichtung für medizinische Geräte hinaus können die erfindungsgemäßen Polyurethanharnstoffe auch für weitere technische Anwendungen im nicht-medizinischen Bereich benutzt werden.

So dienen die erfindungsgemäße Polyurethanharnstoffe zu Herstellung von Beschichtungen als Schutz von Oberflächen vor Beschlagen mit Feuchtigkeit, zur Herstellung von leicht zu reinigenden oder von selbstreinigenden Oberflächen. Diese hydrophilen Beschichtungen verringern auch die Aufnahme von Schmutz und verhindern die Bildung von Wasserflecken. Denkbare Anwendungen im Außenbereich sind beispielsweise Fensterscheiben und Dachluken, Glasfassaden oder Plexiglasdächer. Im Innenbereich können solche Materialien für die Beschichtung von Oberflächen im Sanitärbereich benutzt werden. Weitere Anwendungen sind die Beschichtung von Brillengläsern oder von Verpackungsmaterialien wie Lebensmittelverpackungen zur Vermeidung von Feuchtigkeitsbeschlag oder Tropfenbildung durch kondensiertes Wasser.

Die erfindungsgemäßen Polyurethanharnstoffe eignen sich ebenso zur Ausrüstung von Oberflächen im Kontakt mit Wasser zur Verminderung des Bewuchses. Diesen Effekt nennt man auch Antifoulingeffekt. Eine sehr wichtige Anwendung dieses Antifoulingeffektes ist im Bereich der Unterwasseranstriche von Schiffsrümpfen. Schiffsrümpfe ohne Antifoulingausrüstung werden sehr schnell von Meeresorganismen bewachsen, was durch erhöhte Reibung zu einer Verringerung der möglichen Geschwindigkeit und einem höheren Treibstoffverbrauch führt. Die erfindungsgemäßen Beschichtungsmaterialien reduzieren oder verhindern den Bewuchs mit Meeresorganismen und verhindern die oben beschriebenen Nachteile dieses Bewuchses. Weitere Anwendungen im Bereich der Antifoulingbeschichtungen sind Artikel für die Fischerei wie Fischernetze sowie alle metallischen Substrate im Unterwassereinsatz wie Rohrleitungen, Bohrinseln, Schleusenkammern und -tore etc. Schiffsrümpfe, die mit den erfindungsgemäßen Beschichtungsmaterialien erzeugte Oberflächen insbesondere unterhalb der Wasserlinie aufweisen, besitzen auch einen reduzierten Reibungswiderstand, so dass derartig ausgerüstete Schiffe entweder einen reduzierten Brennstoff-Verbrauch aufweisen oder höhere Geschwindigkeiten erreichen. Dies ist insbesondere im Sportbootbereich und Yachtbau von Interesse.

Ein weiteres wichtiges Anwendungsgebiet der obengenannten hydrophilen Beschichtungsmaterialien ist die Druckindustrie. Hydrophobe Oberflächen können durch die erfindungsgemäßen Beschichtungen hydrophilisiert werden und sind dadurch mit polaren Druckfarben bedruckbar bzw. können mittels Tintenstrahltechnik aufgebracht werden.

Ein weiterer Anwendungsbereich der erfindungsgemäßen hydrophilen Beschichtungen sind Formulierungen für kosmetische Anwendungen.

Beschichtungen aus den erfindungsgemäßen Polyurethanharnstoffen können mittels verschiedener Verfahren aufgebracht werden. Geeignete Beschichtungstechniken sind hierfür beispielsweise Rakeln, Drucken, Transferbeschichten, Sprühen, Spincoating oder Tauchen.

Beschichtet werden können vielerlei Substrate wie Metalle, Textilien, Keramiken und Kunststoffe. Bevorzugt ist die Beschichtung von medizinischen Geräten, die aus Kunststoff oder Metallen gefertigt sind. Als Metalle können beispielsweise genannt werden: Medizinischer Edelstahl und Nickel-Titan-Legierungen. Es sind viele Polymermaterialien denkbar, aus denen das medizinische Geräte aufgebaut sein kann, beispielsweise Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethylen oder Copolymeren aus Polyethylen und Polypropylen; Silikon; Polyvinylchlorid (PVC) und Polyurethanen. Zur besseren Haftung des hydrophilen Polyurethanharnstoffe auf dem medizinischen Gerät können als Untergrund vor dem Auftragen dieser hydrophilen Beschichtungsmaterialien noch weitere geeignete Beschichtungen aufgetragen werden.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethanharnstoffdispersion oder -lösung bei 115 °C bis zur Gewichtskonstanz (15-20 min) mittels eines Infrarottrockners getrocknet.

Die Messung der mittleren Teilchengrößen der Polyurethanharnstoffdispersionen oder -lösung erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene Dispersion oder Lösung.

Die Zugfestigkeiten wurden nach DIN 53504 bestimmt.

Viskositätsmessungen wurden mit dem Physics MCR 51 Rheometer der Firma Anton Paar GmbH, Ostfildern, Deutschland, durchgeführt.

### Verwendete Substanzen und Abkürzungen:

| | |
|---|---|
| Desmophen^{®} C2200: | Polycarbonatpolyol, OH-Zahl 56 mg KOH/g, zahlenmittleres Molekulargewicht 2000 g/mol (Bayer MaterialScience AG, Leverkusen, DE) |
| | |
| Desmophen^{®} C1200: | Polycarbonatpolyol, OH-Zahl 56 mg KOH/g, zahlenmittleres Molekulargewicht 2000 g/mol (Bayer MaterialScience AG, Leverkusen, DE) |
| | |
| Polyether LB 25: | (monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE) |
| | |
| 1,4-Cyclohexandimethanol | Produkt der Fa. Eastman, Kingsport, Tennessee, USA |

### Polycarbonatdiole

### Beispiel 1

Herstellung eines cycloaliphatischen Polycarbonatdiols auf Basis Cyclohexandimethanol mit einem zahlenmittleren Molekulargewicht von ca. 1000 g/mol

In einem 16 1 Druckreaktor mit Destillationsaufsatz, Rührer und Vorlage wurden 3042,4 g Cyclohexandimethanol unter Stickstoffathmosphäre vorgelegt und 2 Stunden bei 90 °C und einem Vakuum von 20 mbar entwässert. Dann wurde mit Stickstoff belüftet und ein Rückflusskühler montiert. Anschließend wurden 0,7 g Yttrium(III)acetylacetonat sowie 2377,9 g Dimethylcarbonat bei 90 °C zugegeben. Unter Stickstoffatmosphäre wurde das Reaktionsgemisch in 2 h auf 135 °C aufgeheizt und unter Rühren 24 h unter Rückfluss gehalten. Danach wurde die Temperatur auf 150 °C erhöht und 4 Stunden bei dieser Temperatur gerührt. Dann wurde die Temperatur auf 180 °C erhöht und weitere 4 Stunden bei dieser Temperatur gerührt. Anschließend wurde auf 130 °C abgekühlt und der Reaktionskessel für die anschließende Destillation umgebaut. Dann wurde das Spaltprodukt Methanol im Gemisch mit Dimethylcarbonat per Destillation entfernt, wobei die Temperatur schrittweise auf 180 °C erhöht wurde. Es wurde dann noch 6 Stunden bei 180 °C und einem Vakuum von 20 mbar. Dabei erfolgte die weitere Entfernung von Methanol im Gemisch mit Dimethylcarbonat aus dem Reaktionsgemisch.

Nach Belüftung und Abkühlung des Reaktionsansatzes auf Raumtemperatur, wurde ein gelbliches, festes Polycarbonatdiol mit folgenden Kennzahlen erhalten:
Mₙ = 984 g/mol; OH-Zahl = 114 mg KOH/g;

### Beispiel 2

Herstellung eines cycloaliphatischen Polycarbonatdiols auf Basis Cyclohexandimethanol mit einem zahlenmittleren Molekulargewicht von ca. 500 g/mol

Vorgehen wie in Beispiel 1, wobei 3119,5 g Cyclohexandimethanol, 0,7 g Yttrium(III)acetylacetonat und 1977,3 g Dimethylcarbonat verwendet wurden.

Man erhielt ein gelbliches, festes Polycarbonatdiol mit folgenden Kennzahlen: Mₙ = 524 g/mol; OH-Zahl = 214 mg KOH/g.

### Polyurethanharnstofflösungen:

### Beispiel 3a (Vergleich)

195,4 g Desmophen C 2200, 30,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 11,8 g Isophorondiamin in 94,0 g 1-Methoxypropanol-2 zugegeben. Nach beendetem Aufbau des Molgewichtes und Erreichen des gewünschten Viskositätsbereiches rührte man weitere 5 Stunden bei Raumtemperatur, um den restlichen Isocyanatgehalt mit iso-Propanol zu blockieren. Man erhielt 929 g einer 31,9%igen Polyurethanharnstofflösung in Toluol/iso-Propanol/1-Methoxypropanol-2 mit einer Viskosität von 37100 mPas bei 22 °C.

### Beispiel 4a (erfindungsgemäß)

97,8 g Desmophen C 2200, 48,9 g Polycarbonatdiol des Beispiels 1, 30,0 g LB 25 und 47,8 g 4,4'-Bis(isocyanatocyclohexyl)methan (H₁₂MDI) wurden bei 110 °C bis zu einem konstanten NCO-Gehalt von 2,8 % umgesetzt. Man ließ abkühlen und verdünnte mit 320,0 g Toluol und 185 g iso-Propanol. Bei Raumtemperatur wurden eine Lösung von 12,3 g Isophorondiamin in 100 g 1-Methoxypropanol-2 zugegeben. Nach beendetem Aufbau des Molgewichtes und Erreichen des gewünschten Viskositätsbereiches rührte man weitere 16 Stunden bei Raumtemperatur, um den restlichen Isocyanatgehalt mit iso-Propanol zu blockieren. Man erhielt 841,8 g einer 28,6%igen Polyurethanharnstofflösung in Toluol/iso-Propanol/1-Methoxypropanol-2 mit einer Viskosität von 14100 mPas bei 22 °C.

### Beispiel 5a (erfindungsgemäß)

146,8 g Desmophen C 2200, 24,5 g Polycarbonatdiol des Beispiels 1, 30,0 g LB 25 und 47,8 g 4,4'-Bis(isocyanatocyclohexyl)methan (H₁₂MDI) wurden bei 110 °C bis zu einem konstanten NCO-Gehalt von 2,6 % umgesetzt. Man ließ abkühlen und verdünnte mit 320,0 g Toluol und 185 g iso-Propanol. Bei Raumtemperatur wurden eine Lösung von 11,4 g Isophorondiamin in 90 g 1-Methoxypropanol-2 zugegeben. Nach beendetem Aufbau des Molgewichtes und Erreichen des gewünschten Viskositätsbereiches rührte man weitere 22 Stunden bei Raumtemperatur, um den restlichen Isocyanatgehalt mit iso-Propanol zu blockieren. Man erhielt 855,5 g einer 30,9%igen Polyurethanharnstofflösung in Toluol/iso-Propanol/1-Methoxypropanol-2 mit einer Viskosität von 24400 mPas bei 22 °C.

### Beispiel 6a Kontaktwinkel und 100%-Module von Vergleichsbeispiel 3a gegen erfindungsgemäße Beispiele 4a und 5a

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g organischer 15%iger Polyurethanlösung homogen bedeckt Alle organischen Polyurethanlösungen wurden mit einem Lösungsmittelgemisch aus 65 Gew% Toluol und 35 Gew% iso-Propanol auf einen Polymergehalt von 15 Gew% verdünnt. Durch Rotation des Probentellers für 20 sec bei 1300 Umdrehungen pro Minute erhielt man eine homogene Beschichtung, die 1 h bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wurde eine statische Kontaktwinkelmessung durchgeführt. Mittels des Video- Randwinkelmessgerätes OCA20 der Firma Dataphysics mit rechnergesteuerten Spritzen wurde auf das Muster 10 Tropfen Milliporewasser aufgesetzt und deren statischer Benetzungsrandwinkel gemessen. Zuvor wurde mittels eines Antistatikföns die statische Aufladung (falls vorhanden) auf der Probenoberfläche entfernt.

### 2. Herstellung der Beschichtungen für die Messung des 100%-Moduls

Auf Trennpapier werden mit einem 200µm-Rakel Schichten hergestellt, die 15 min bei 100 °C getrocknet werden. Ausgestanzte Formkörper werden nach DIN 53504 untersucht.

### 3. Untersuchungsergebnisse

**Tabelle 1: Kontaktwinkel und 100%-Module der Filme aus Materialien der Beispiele 5a-11a**

| Beispiel-Nr. | Kontaktwinkel (°) | 100%-Modul (N/mm²) |
|---|---|---|
| Vergleichsbeispiel 3a | 21 | 2,3 |
| Beispiel 4a | 22 | 7,9 |
| Beispiel 5a | 26 | 4,1 |

Die erfindungsgemäßen Beispiele 4a und 5a unterscheiden sich dadurch, dass im Vergleich zum Vergleichsbeispiel 3a ein Teil des Polycarbonatdiols Desmophen C2200 durch das erfindungswesentliche Polycarbonatdiol ersetzt wurde. Die Materialien zeigen als Beschichtung ähnlich hydrophile Eigenschaften wie das Vergleichsbeispiel 3a. Die 100%-Module sind alle höher als dasjenige des Vergleichsbeispiels 3a.

### Beispiel 7a Vergleichsbeispiel

Dieses Beispiel beschreibt die Herstellung eines Vergleichsbeispiels einer Polyurethanharnstoff-Lösung.

195,4 g Desmophen C 2200, 40,0 g LB 25 und 47,8 g 4,4'-Bis(isocyanatocyclohexyl)methan (H₁₂MDI) wurden bei 110 °C bis zu einem konstanten NCO-Gehalt von 2,2 % umgesetzt. Man ließ abkühlen und verdünnte mit 350,0 g Toluol und 200 g iso-Propanol. Bei Raumtemperatur wurden eine Lösung von 12,0 g Isophorondiamin in 100,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 945 g einer 31,6%igen Polyurethanharnstofflösung in Toluol/iso-Propanol/1-Methoxypropanol-2 mit einer Viskosität von 19300 mPas bei 22 °C.

### Beispiel 8a (erfindungsgemäß)

97,5 g Desmophen C 2200, 48,9 g Polycarbonatdiol des Beispiels 1, 40,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt. Man ließ abkühlen und verdünnte mit 320,0 g Toluol und 180 g iso-Propanol. Bei Raumtemperatur wurden eine Lösung von 11,2 g Isophorondiamin in 100 g 1-Methoxypropanol-2 zugegeben. Nach beendetem Aufbau des Molgewichtes und Erreichen des gewünschten Viskositätsbereiches rührte man weitere 5 Stunden bei Raumtemperatur, um den restlichen Isocyanatgehalt mit iso-Propanol zu blockieren. Man erhielt 845,4 g einer 29,5%igen Polyurethanharnstofflösung in Toluol/iso-Propanol/1-Methoxypropanol-2 mit einer Viskosität von 13100 mPas bei 22 °C.

### Beispiel 9a (erfindungsgemäß)

146,8 g Desmophen C 2200, 24,5 g Polycarbonatdiol des Beispiels 1, 40,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 320,0 g Toluol und 180 g iso-Propanol. Bei Raumtemperatur wurden eine Lösung von 11,5 g Isophorondiamin in 97,9 g 1-Methoxypropanol-2 zugegeben. Nach beendetem Aufbau des Molgewichtes und Erreichen des gewünschten Viskositätsbereiches rührte man weitere 22 Stunden bei Raumtemperatur, um den restlichen Isocyanatgehalt mit iso-Propanol zu blockieren. Man erhielt 868,5 g einer 31,6%igen Polyurethanharnstofflösung in Toluol/iso-Propanol/1-Methoxypropanol-2 mit einer Viskosität von 32800 mPas bei 22 °C.

### Beispiel 10a (erfindungsgemäß)

97,5 g Desmophen C 2200, 25,6 g Polycarbonatdiol des Beispiels 2, 40,0 g LB 25 und 47,8 g 4,4'-Bis(isocyanatocyclohexyl)methan (H₁₂MDI) wurden bei 110 °C bis zu einem konstanten NCO-Gehalt von 3,0 % umgesetzt. Man ließ abkühlen und verdünnte mit 310,0 g Toluol und 170 g iso-Propanol. Bei Raumtemperatur wurden eine Lösung von 12,7 g Isophorondiamin in 100 g 1-Methoxypropanol-2 zugegeben. Nach beendetem Aufbau des Molgewichtes und Erreichen des gewünschten Viskositätsbereiches rührte man weitere 22 Stunden bei Raumtemperatur, um den restlichen Isocyanatgehalt mit iso-Propanol zu blockieren. Man erhielt 803,6 g einer 28,2%igen Polyurethanharnstofflösung in Toluol/iso-Propanol/1-Methoxypropanol-2 mit einer Viskosität von 8600 mPas bei 22 °C.

### Beispiel 11a (erfindungsgemäß)

146,8 g Desmophen C 2200, 12,8 g Polycarbonatdiol des Beispiels 2, 40,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt. Man ließ abkühlen und verdünnte mit 330,0 g Toluol und 185 g iso-Propanol. Bei Raumtemperatur wurden eine Lösung von 11,7 g Isophorondiamin in 100 g 1-Methoxypropanol-2 zugegeben. Nach beendetem Aufbau des Molgewichtes und Erreichen des gewünschten Viskositätsbereiches rührte man weitere 6 Stunden bei Raumtemperatur, um den restlichen Isocyanatgehalt mit iso-Propanol zu blockieren. Man erhielt 874,1 g einer 30,0%igen Polyurethanharnstofflösung in Toluol/iso-Propanol/1-Methoxypropanol-2 mit einer Viskosität von 1 1400 mPas bei 22 °C.

### Beispiel 12a Kontaktwinkel und 100%-Module von Vergleichsbeispiel 7a gegen erfindungsgemäße Beispiele 8a-11a

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g organischer 15%iger Polyurethanharnstofflösung homogen bedeckt. Alle organischen Polyurethanharnstofflösungen wurden mit einem Lösungsmittelgemisch aus 65 Gew% Toluol und 35 Gew% iso-Propanol auf einen Polymergehalt von 15 Gew% verdünnt. Durch Rotation des Probentellers für 20 sec bei 1300 Umdrehungen pro Minute erhielt man eine homogene Beschichtung, die 1 h bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wurde eine statische Kontaktwinkelmessung durchgeführt. Mittels des Video- Randwinkelmessgerätes OCA20 der Firma Dataphysics mit rechnergesteuerten Spritzen wurde auf das Muster 10 Tropfen Milliporewasser aufgesetzt und deren statischer Benetzungsrandwinkel gemessen. Zuvor wurde mittels eines Antistatikföns die statische Aufladung (falls vorhanden) auf der Probenoberfläche entfernt.

### 2. Herstellung der Beschichtungen für die Messung des 100%-Moduls

Auf Trennpapier werden mit einem 200µm-Rakel Schichten hergestellt, die 1 min bei 100 °C getrocknet werden. Ausgestanzte Formkörper werden nach DIN 53504 untersucht.

### 3. Untersuchungsergebnisse

**Tabelle 2: Kontaktwinkel und 100%-Module der Filme aus Materialien der Beispiele 7a-11a**

| Beispiel-Nr. | Kontaktwinkel (°) | 100%-Modul (N/mm²) |
|---|---|---|
| Vergleichsbeispiel 7a | 14 | 2,7 |
| Beispiel 8a | 19 | 5,6 |
| Beispiel 9a | 22 | 3,5 |
| Beispiel 10a | 26 | 6,2 |
| Beispiel 11a | 15 | 3,9 |

Die erfindungsgemäßen Beispiele 8a bis 11a unterscheiden sich dadurch, dass im Vergleich zum Vergleichsbeispiel 7a ein Teil des Polycarbonatdiols Desmophen C2200 durch das erfindungswesentliche Polycarbonatdiol ersetzt wurde. Die Materialien zeigen als Beschichtung ähnlich hydrophile Eigenschaften wie das Vergleichsbeispiel 7a. Die 100%-Module sind alle höher als dasjenige des Vergleichsbeispiels 7a.

### Polyurethanharnstoffdispersionen

### Beispiel 3b (Vergleich)

277,2 g Desmophen C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 16 h war der theoretische NCO Wert von 2,4% erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethanharnstoffdispersion mit einem Festkörpergehalt von 40,7 % und einer mittleren Teilchengröße von 136 nm erhalten.

### Beispiel 4b (erfindungsgemäß)

138,6 g Desmophen C 2200, 69,3 g Polycarbonatdiol des Beispiels 1, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 75 min war der theoretische NCO Wert von 2,9 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 530 g Wasser dispergiert. Es folgte die Entfernung des Lösungsmittels durch Destillation im Vakuum. Es wurde eine lagerstabile Polyurethanharnstoffdispersion mit einem Festkörpergehalt von 38,4 % und einer mittleren Teilchengroße von 143 nm erhalten.

### Beispiel 5b (erfindungsgemäß)

208 g Desmophen C 2200, 34,7 g Polycarbonatdiol des Beispiels 1, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 16 h war der theoretische NCO Wert von 2,6 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 550 g Wasser dispergiert. Es folgte die Entfernung des Lösungsmittels durch Destillation im Vakuum. Es wurde eine lagerstabile Polyurethanharnstoffdispersion mit einem Festkörpergehalt von 43,0 % und einer mittleren Teilchengröße von 115 nm erhalten.

### Beispiel 6b (erfindungsgemäß)

138,6 g Desmophen C 2200, 36,2 g Polycarbonatdiol des Beispiels 2, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 14,5 h war der theoretische NCO Wert von 3,3 % erreicht. Das fertige Prepolymer wurde bei 50 °C in 670 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 500 g Wasser dispergiert. Es folgte die Entfernung des Lösungsmittels durch Destillation im Vakuum. Es wurde eine lagerstabile Polyurethanharnstoffdispersion mit einem Festkörpergehalt von 38,6 % und einer mittleren Teilchengröße von 147 nm erhalten.

### Beispiel 7b (erfindungsgemäß)

208 g Desmophen C 2200, 18,1 g Polycarbonatdiol des Beispiels 2, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 17 h war der theoretische NCO Wert von 2,7 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 550 g Wasser dispergiert. Es folgte die Entfernung des Lösungsmittels durch Destillation im Vakuum. Es wurde eine lagerstabile Polyurethanharnstoffdispersion mit einem Festkörpergehalt von 39,7 % und einer mittleren Teilchengröße von 122 nm erhalten.

### Beispiel 8b Kontaktwinkel und 100 %-Module von Vergleichsbeispiel 3b gegen erfindungsgemäße Beispiele 4b-7b

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g wässriger unverdünnter Polyurethanharnstoffdispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wird eine statische Kontaktwinkelmessung durchgeführt. Mittels des Video- Randwinkelmessgerätes OCA20 der Firma Dataphysics mit rechnergesteuerten Spritzen wird auf das Muster 10 Tropfen Milliporewasser aufgesetzt und deren statischer Benetzungsrandwinkel gemessen. Zuvor wird mittels eines Antistatikföns die statische Aufladung (falls vorhanden) auf der Probenoberfläche entfernt.

### 2. Herstellung der Beschichtungen für die Messung des 100%-Moduls

Die Beschichtungen wurden auf Trennpapier mit einem 200 µm-Rakel aufgetragen. Vor der Filmherstellung werden die wässrigen Dispersionen mit 2 Gew% eines Verdickers (Borchi Gel A LA, Fa. Borchers, Langenfeld, Deutschland) versetzt und 30 min bei RT durch Rühren homogenisiert. Die feuchten Filme wurden 15 min bei 100 °C getrocknet.

Ausgestanzte Formkörper wurden gemäß DIN 53504 untersucht.

### 3. Untersuchungsergebnisse

**Tabelle 1: Kontaktwinkel und 100%-Module der Filme aus Materialien der Beispiele 3b-7b**

| Beispiel-Nr. | Kontaktwinkel (°) | 100%-Modul (N/mm²) |
|---|---|---|
| Vergleichsbeispiel 3b | 10 | 2,6 |
| Beispiel 4b | 16 | 9,4 |
| Beispiel 5b | < 10 | 3,8 |
| Beispiel 6b | 22 | 8,2 |
| Beispiel 7b | 16 | 3,3 |

Die erfindungsgemäßen Beispiele 4b bis 7b unterscheiden sich dadurch, dass im Vergleich zum Vergleichsbeispiel 4b ein Teil des Polycarbonatdiols Desmophen C2200 durch ein neues erfindungsgemäßes Polycarbonatdiol ersetzt wurde. Die Materialien zeigen als Beschichtung ähnlich hydrophile Eigenschaften wie das Vergleichsbeispiel 3b, immer kleinere Kontaktwinkel als 30 °. Die 100%-Module sind alle höher als dasjenige des Vergleichsbeispiels 3b.

### Beispiel 9b Vergleichsbeispiel

277,2 g Desmophen C 1200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 75 min war der theoretische NCO-Wert von 2,4 % erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethanharnstoffdispersion mit einem Festkörpergehalt von 39,9 % und einer mittleren Teilchengröße von 169 nm erhalten.

### Beispiel 10b (erfindungsgemäß)

208,0 g Desmophen C 1200, 34,7 g Polycarbonatdiol des Beispiels 1, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2 h 40 min war der theoretische NCO Wert von 2,6 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 530 g Wasser dispergiert. Es folgte die Entfernung des Lösungsmittels durch Destillation im Vakuum. Es wurde eine lagerstabile Polyurethanharnstoffdispersion mit einem Festkörpergehalt von 40,8 % und einer mittleren Teilchengröße von 132 nm erhalten.

### Beispiel 11b (erfindungsgemäß)

138,6 g Desmophen C 1200, 69,3 g Polycarbonatdiol des Beispiels 1, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2,5 h war der theoretische NCO Wert von 2,9 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 530 g Wasser dispergiert. Es folgte die Entfernung des Lösungsmittels durch Destillation im Vakuum. Es wurde eine lagerstabile Polyurethanharnstoffdispersion mit einem Festkörpergehalt von 40,2 % und einer mittleren Teilchengröße von 132 nm erhalten.

### Beispiel 12b (erfindungsgemäß)

138,6 g Desmophen C 1200, 36,2 g Polycarbonatdiol des Beispiels 2, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2,5 h war der theoretische NCO Wert von 3,2 % erreicht. Das fertige Prepolymer wurde bei 50 °C in 670 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 500 g Wasser dispergiert. Es folgte die Entfernung des Lösungsmittels durch Destillation im Vakuum. Es wurde eine lagerstabile Polyurethanharnstoffdispersion mit einem Festkörpergehalt von 39,7 % und einer mittleren Teilchengröße von 151 nm erhalten.

### Beispiel 13b Kontaktwinkel und 100 %-Module von Vergleichsbeispiel 9b gegen erfindungsgemäße Beispiele 10b-12b

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g wässriger unverdünnter Polyurethanharnstoffdispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wird eine statische Kontaktwinkelmessung durchgeführt. Mittels des Video- Randwinkelmessgerätes OCA20 der Firma Dataphysics mit rechnergesteuerten Spritzen wird auf das Muster 10 Tropfen Milliporewasser aufgesetzt und deren statischer Benetzungsrandwinkel gemessen. Zuvor wird mittels eines Antistatikföns die statische Aufladung (falls vorhanden) auf der Probenoberfläche entfernt.

### 2. Herstellung der Beschichtungen für die Messung des 100%-Moduls

Die Beschichtungen wurden auf Trennpapier mit einem 200 µm-Rakel aufgetragen. Vor der Filmherstellung werden die wässrigen Dispersionen mit 2 Gew% eines Verdickers (Borchi Gel A LA, Fa. Borchers, Langenfeld, Deutschland) versetzt und 30 min bei RT durch Rühren homogenisiert. Die feuchten Filme wurden 15 min bei 100 °C getrocknet.

Ausgestanzte Formkörper wurden gemäß DIN 53504 untersucht.

### 3. Untersuchungsergebnisse

**Tabelle 2: Kontaktwinkel und 100%-Module der Filme aus Materialien der Beispiele 9b-12b**

| Beispiel-Nr. | Kontaktwinkel (°) | 100%-Modul (N/mm²) |
|---|---|---|
| Vergleichsbeispiel 9b | 14 | 1,6 |
| Beispiel 10b | 23 | 2,7 |
| Beispiel 11b | 22 | 4,7 |
| Beispiel 12b | 26 | 3,9 |

Die erfindungsgemäßen Beispiele 10b bis 12b unterscheiden sich dadurch, dass im Vergleich zum Vergleichsbeispiel 9b ein Teil des Polycarbonatdiols Desmophen C1200 durch ein neues erfindungsgemäßes Polycarbonatdiol ersetzt wurde. Die Materialien zeigen als Beschichtung ähnlich hydrophile Eigenschaften wie das Vergleichsbeispiel 9b, immer kleinere Kontaktwinkel als 30 °. Die 100%-Module sind alle höher als dasjenige des Vergleichsbeispiels 9b.

### Beispiel 14b Vergleichsbeispiel

269,8 g Desmophen C 2200, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21 h war der theoretische NCO-Wert von 2,4 % erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethanharnstoffdispersion mit einem Festkörpergehalt von 41,3 % und einer mittleren Teilchengröße von 109 nm erhalten.

### Beispiel 15b (erfindungsgemäß)

135 g Desmophen C 2200, 67,5 g Polycarbonatdiol des Beispiels 1, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 17,5 h war der theoretische NCO Wert von 2,5 % erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 520 g Wasser dispergiert. Es folgte die Entfernung des Lösungsmittels durch Destillation im Vakuum. Es wurde eine lagerstabile Polyurethanharnstoffdispersion mit einem Festkörpergehalt von 39,8 % und einer mittleren Teilchengröße von 80 nm erhalten.

### Beispiel 16b (erfindungsgemäß)

202,4 g Desmophen C 2200, 33,7 g Polycarbonatdiol des Beispiels 1, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 17 h war der theoretische NCO Wert von 2,5 % erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 540 g Wasser dispergiert. Es folgte die Entfernung des Lösungsmittels durch Destillation im Vakuum. Es wurde eine lagerstabile Polyurethanharnstoffdispersion mit einem Festkörpergehalt von 41,3 % und einer mittleren Teilchengröße von 85 nm erhalten.

### Beispiel 17b (erfindungsgemäß)

202,6 g Desmophen C 2200, 17,7 g Polycarbonatdiol des Beispiels 2, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 17 h war der theoretische NCO Wert von 2,6 % erreicht. Das fertige Prepolymer wurde bei 50 °C in 670 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 550 g Wasser dispergiert. Es folgte die Entfernung des Lösungsmittels durch Destillation im Vakuum. Es wurde eine lagerstabile Polyurethanharnstoffdispersion mit einem Festkörpergehalt von 41,4 % und einer mittleren Teilchengröße von 85 nm erhalten.

### Beispiel 18b (erfindungsgemäß)

135 g Desmophen C 2200, 35,3 g Polycarbonatdiol des Beispiels 2, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 17,5 h war der theoretische NCO Wert von 3,1 % erreicht. Das fertige Prepolymer wurde bei 50 °C in 620 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 490 g Wasser dispergiert. Es folgte die Entfernung des Lösungsmittels durch Destillation im Vakuum. Es wurde eine lagerstabile Polyurethanharnstoffdispersion mit einem Festkörpergehalt von 39,2 % und einer mittleren Teilchengröße von 79 nm erhalten.

### Beispiel 19b Kontaktwinkel und 100 %-Module von Vergleichsbeispiel 14b gegen erfindungsgemäße Beispiele 15b-18b

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g wässriger unverdünnter Polyurethandispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wird eine statische Kontaktwinkelmessung durchgeführt. Mittels des Video- Randwinkelmessgerätes OCA20 der Firma Dataphysics mit rechnergesteuerten Spritzen wird auf das Muster 10 Tropfen Milliporewasser aufgesetzt und deren statischer Benetzungsrandwinkel gemessen. Zuvor wird mittels eines Antistatikföns die statische Aufladung (falls vorhanden) auf der Probenoberfläche entfernt.

### 2. Verstellung der Beschichtungen für die Messung des 100%-Moduls

Die Beschichtungen wurden auf Trennpapier mit einem 200 µm-Rakel aufgetragen. Vor der Filmherstellung werden die wässrigen Dispersionen mit 2 Gew% eines Verdickers (Borchi Gel A LA, Fa. Borchers, Langenfeld, Deutschland) versetzt und 30 min bei RT durch Rühren homogenisiert. Die feuchten Filme wurden 15 min bei 100 °C getrocknet.

Ausgestanzte Formkörper wurden gemäß DIN 53504 untersucht.

### 3. Untersuchungsergebnisse

**Tabelle 3: Kontaktwinkel und 100%-Module der Filme aus Materialien der Beispiele 14b-18b**

| Beispiel-Nr. | Kontaktwinkel (°) | 100%-Modul (N/mm²) |
|---|---|---|
| Vergleichsbeispiel 14b | 11 | 1,9 |
| Beispiel 15b | 13 | 5,0 |
| Beispiel 16b | <10 | 2,8 |
| Beispiel 17b | <10 | 2,9 |
| Beispiel 18b | 17 | 4,4 |

Die erfindungsgemäßen Beispiele 15b bis 18b unterscheiden sich dadurch, dass im Vergleich zum Vergleichsbeispiel 14b ein Teil des Polycarbonatdiols Desmophen C2200 durch ein neues erfindungsgemäßes Polycarbonatdiol ersetzt wurde. Die Materialien zeigen als Beschichtung ähnlich hydrophile Eigenschaften wie das Vergleichsbeispiel 14b, immer kleinere Kontaktwinkel als 30 °. Die 100%-Module sind alle höher als dasjenige des Vergleichsbeispiels 14b.

## Patentansprüche

1. Polyurethanharnstoff, der mit wenigstens einer Copolymereinheit aus Polyethylenoxid und Polypropylenoxid terminiert ist, **dadurch gekennzeichnet, dass** der Polyurethanharnstoff wenigstens eine Struktureinheit der Formel (I) 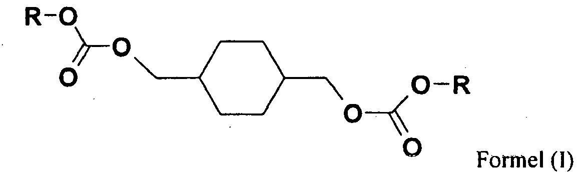 aufweist, die über wenigstens eine Bindungen R mit der Polymerkette verknüpft ist.

2. Polyurethanharnstoff nach Anspruch 1, **dadurch gekennzeichnet, dass** er frei von ionischen oder ionogenen Gruppen ist.

3. Polyurethanharnstoff nach einem der Ansprüche 1 oder 2, **dadurch gekennzeichnet, dass** er auf einer Polycarbonatpolyolkomponente basiert, die bevorzugt eine mittlere Hydroxylfunktionalität von 1,7 bis 2,3 aufweist.

4. Polyurethanharnstoff nach Anspruch 3, **dadurch gekennzeichnet, dass** die Polycarbonatpolyolkomponente Polycarbonatpolyole a1) umfasst, die durch Umsetzung von Kohlensäurederivaten mit difunktionellen Alkoholen der Formel (II) 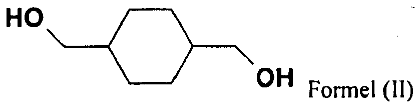 erhältlich sind.

5. Polyurethanharnstoff nach Anspruch 4, **dadurch gekennzeichnet, dass** die Polycarbonatpolyolkomponente neben den Polycarbonatpolyolen a1) weitere Polycarbonatpolyole a2) umfasst.

6. Polyurethanharnstoff nach Anspruch 5, **dadurch gekennzeichnet, dass** es sich bei den Polycarbonatpolyolen a2) um Verbindungen mit einer mittleren Hydroxylfunktionalität von 1,7 bis 2,3 und einem durch die OH-Zahl bestimmten Molekulargewicht von 400 bis 6000 g/mol auf Basis von Hexandiol-1,6, Butandiol-1,4 oder deren Mischungen handelt.

7. Polyurethanharnstoff nach einem der Ansprüche 1 bis 6, **dadurch gekennzeichnet, dass** die zur Terminierung eingesetzte Copolymereinheit aus Polyethylenoxid und Polypropylenoxid auf einem monohydroxyfunktionellen gemischten Polyalkylenoxidpolyether aus mindestens 40 mol-% Ethylenoxid- und maximal 60 mol-% Propylenoxideinheiten, bezogen auf den Gesamtanteil an Alkylenoxideinheiten, mit einem zahlenmittleren Molekulargewicht von 500 g/mol bis 5000 g/mol basiert.

8. Polyurethanharnstoff, nach einem der Ansprüche 1 bis 7 **dadurch gekennzeichnet, dass** er in gelöster Form vorliegt.

9. Polyurethanharnstoff nach Anspruch 8, **dadurch gekennzeichnet, dass** er in Dimethylformamid, N-Methylacetamid, Tetramethylharnstoff, N-Methylpyrrolidon, Toluol, lineare und cyclische Ester, Ether, Ketone und / oder Alkohol und bevorzugt in Mischungen aus Toluol und Ethanol, n-Propanol, iso-Propanol und/oder 1-Methoxy-2-propanol gelöst ist.

10. Polyurethanharnstoff nach einem der Ansprüche 1 bis 7, **dadurch gekennzeichnet, dass** er in dispergierter Form vorliegt.

11. Polyurethanharnstoff nach Anspruch 10, **dadurch gekennzeichnet, dass** er in Wasser dispergiert ist.

12. Polyurethanharnstoff nach einem der Ansprüche 1 bis 11, **dadurch gekennzeichnet, dass** er wenigstens einen pharmakologischen Wirkstoff umfasst.

13. Verwendung eines Polyurethanharnstoffs nach einem der Ansprüche 1 bis 12 zur Herstellung einer Beschichtung auf einem Substrat.

14. Verwendung nach Anspruch 13, **dadurch gekennzeichnet, dass** das Substrat ein medizinisches Gerät, insbesondere ein Stent oder ein Katheter ist.

15. Verfahren zur Herstellung eines Polyurethanharnstoffs nach einem der Ansprüche 1 bis 12, bei dem eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente miteinander umgesetzt werden.

## Claims

1. Polyuxethaneurea which is terminated with at least one copolymer unit of polyethylene oxide and polypropylene oxide, **characterized in that** the polyuxethaneurea has at least one structual unit of the formula (I) 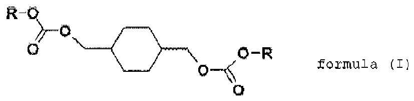 which is linked to the polymer chain by at least one bond R.

2. Polyurethaneurea according to Claim 1, **characterized in that** it is free from ionic or ionogenic groups.

3. Polyurethaneurea according to either of Claims 1 and 2, **characterized in that** it is based on a polycarbonate polyol component, which preferably has an average hydroxyl functionality of 1.7 to 2.3.

4. Polyurethaneurea according to Claim 3, **characterized in that** polycarbonate polyol component comprises polycarbonate polyols a1) which are obtainable by reacting carbonic acid derivatives with difunctional alcohols of the formula (II)

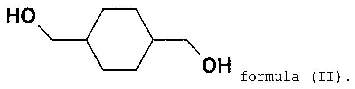

5. Polyurethaneurea according to Claim 4, **characterized in that** polycarbonate polyol component comprises, in addition to the polycarbonate polyols a1), further polycarbonate polyols a2).

6. Polyurethaneurea according to Claim 5, **characterized in that** the polycarbonate polyols a2) are compounds having an average hydroxyl functionality of 1.7 to 2.3 and a molecular weight, as determined by the OH number of 400 to 6000 g/mol, based on hexane-1,6-diol, butane-1,4-diol or mixtures thereof.

7. Polyurethaneurea according to any of Claims 1 to 6, **characterized in that** the copolymer unit of polyethylene oxide and polypropylene oxide that is used for termination is based on a monohydroxy-functional mixed polyalkylene oxide polyether of at least 40 mol% ethylene oxide units and not more than 60 mol% propylene oxide units based on the total fraction of alkylene oxide units with a number-average molecular weight of 500 g/mol to 5000 g/mol.

8. Polyurethaneurea according to any of Claims 1 to 7, **characterized in that** it is present in dissolved form.

9. Polyurethaneurea according to Claim 8, **characterized in that** it is in solution in dimethylformamide, N-methylacetamide, tetramethylurea, N-methylpyrrolidone, toluene, linear and cyclic esters, ethers, ketones and/or alcohol and preferably in mixtures of toluene and ethanol, n-propanol, isopropanol and/or 1-methoxy-2-propanol.

10. Polyurethaneurea according to any of Claims 1 to 7, **characterized in that** it is present in dispersed form.

11. Polyurethaneurea according to Claim 10, **characterized in that** it is in dispersion in water.

12. Polyurethaneurea according to any of Claims 1 to 11, **characterized in that** it comprises at least one active pharmacological ingredient.

13. Use of a polyurethaneurea according to any of Claims 1 to 12 for producing a coating on a substrate.

14. Use according to Claim 13, **characterized in that** the substrate is a medical device, more particularly a stent or a catheter.

15. Process for preparing a polyurethaneurea according to any of Claims 1 to 12, in which a polycarbonate polyol component a), at least one polyisocyanate component b), at least one polyoxyalkylene ether component c), at least one diamine and/or amino alcohol component d) and, if desired, a further polyol component are reacted with one another.

## Revendications

1. Polyuréthane-urée, qui est terminée par au moins une unité copolymère de poly(oxyde d'éthylène) et de poly(oxyde de propylène), **caractérisée en ce que** la polyuréthane-urée présente au moins une unité de structure de formule (I) 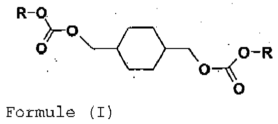 qui est liée via au moins une des liaisons R à la chaîne polymère.

2. Polyuréthane-urée selon la revendication 1, **caractérisée en ce qu'**elle est exempte de groupes ioniques ou ionogènes.

3. Polyuréthane-urée selon l'une quelconque des revendications 1 ou 2, **caractérisée en ce qu'**elle est à base d'un composant polycarbonatepolyol, qui présente de préférence une fonctionnalité hydroxyle moyenne de 1,7 à 2,3.

4. Polyuréthane-urée selon la revendication 3, **caractérisée en ce que** le composant polycarbonatepolyol comprend des polycarbonatepolyols a1) qui peuvent être obtenus par transformation de dérivés d'acide carbonique avec des alcools difonctionnels de formule (II)

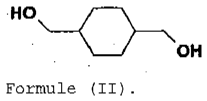

5. Polyuréthane-urée selon la revendication 4, **caractérisée en ce que** le composant polycarbonatepolyol comprend, outre les polycarbonatepolyols a1), d'autres polycarbonatepolyols a2).

6. Polyuréthane-urée selon la revendication 5, **caractérisée en ce qu'**il s'agit, pour les polycarbonatepolyols a2), de composés présentant une fonctionnalité hydroxyle moyenne de 1,7 à 2,3 et un poids moléculaire déterminé par l'indice d'OH de 400 à 6000 g/mole à base d'hexanediol-1,6, de butanediol-1,4 ou leurs mélanges.

7. Polyuréthane-urée selon l'une quelconque des revendications 1 à 6, **caractérisée en ce que** l'unité copolymère de poly(oxyde d'éthylène) et de poly(oxyde de propylène) utilisée pour la terminaison est à base d'un poly(oxyde d'alkylène)-polyéther mixte à fonctionnalité monohydroxy constitué par au moins 40% en mole d'unités d'oxyde d'éthylène et au maximum 60% en mole d'unités d'oxyde de propylène par rapport à la proportion totale d'unités d'oxyde d'alkylène, présentant un poids moléculaire numérique moyen de 500 g/mole à 5000 g/mole.

8. Polyuréthane-urée selon l'une quelconque des revendications 1 à 7, **caractérisée en ce qu'**elle se trouve sous forme dissoute.

9. Polyuréthane-urée selon la revendication 8, **caractérisée en ce qu'**elle est dissoute dans du diméthylformamide, du N-méthylacétamide, de la tétraméthylurée, de la N-méthylpyrrolidone, du toluène, des esters linéaires et cycliques, des éthers, des cétones et/ou des alcools et de préférence dans des mélanges de toluène et d'éthanol, de n-propanol, d'isopropanol et/ou de 1-méthoxy-2-propanol.

10. Polyuréthane-urée selon l'une quelconque des revendications 1 à 7, **caractérisée en ce qu'**elle se trouve sous forme dispersée.

11. Polyuréthane-urée selon la revendication 10, **caractérisée en ce qu'**elle est dispersée dans l'eau.

12. Polyuréthane-urée selon l'une quelconque des revendications 1 à 11, **caractérisée en ce qu'**elle comprend au moins une substance active pharmacologique.

13. Utilisation d'une polyuréthane-urée selon l'une quelconque des revendications 1 à 12 pour la réalisation d'un revêtement sur un substrat.

14. Utilisation selon la revendication 13, **caractérisée en ce que** le substrat est un appareil médical, en particulier une endoprothèse ou un cathéter.

15. Procédé pour la préparation d'une polyuréthane-urée selon l'une quelconque des revendications 1 à 12, dans lequel un composant polycarbonatepolyol a), au moins un composant polyisocyanate b), au moins un composant polyoxyalkylène-éther c), au moins un composant diamine et/ou aminoalcool d) et le cas échéant un autre composant polyol sont transformés les uns avec les autres.
